# EUROPEAN PATENT APPLICATION

(11) **EP 3 981 325 A1**
(43) Date of publication of application: **13.04.2022**
(21) Application number: 20306192.4
(22) Date of filing: 09.10.2020
(51) Int. Cl.: A61B 5/024, A61B 5/022, A61B 5/0225, A61B 5/00, A61B 5/0215

(54) **WEARABLE VITAL SIGNS MONITORING DEVICE AND METHOD**

(71) Applicant: Hinlab, 92200 Neuilly-Sur-Seine (FR)
(72) Inventor: DE BEGON DE LAROUZIERE DE MONTLOSIER, Denys-Michel, 78000 Versailles (FR); PEIRS, Aymeric, 75116 Paris (FR)
(74) Representative: Icosa

(57) **Abstract**

The present invention relates to a wearable device for blood pressure monitoring comprising an armband with an inner surface and an outer surface, the armband comprising: an inflatable bladder; pumping means for inflating and deflating said bladder, the pumping means comprising a pump, a pipe in fluid communication with the inflatable bladder and a valve; a power supply device; a sensor array, the sensor array comprising an optical sensor and a pressure sensor; a processing unit; wherein the armband comprises an inner surface, an outer surface and an opening configured to expose at least partially the optic sensor at the inner surface of the armband.

## Description

### FIELD OF INVENTION

The present invention pertains to the field of devices and methods for physiological parameter estimation. In particular, the invention relates to a wearable device for vital signs monitoring, such as blood pressure monitoring.

### BACKGROUND OF INVENTION

A non-invasive and accurate blood pressure estimation of a subject is needed in hospital and home settings. In some cases, a continuous or regular (e.g. hourly) blood pressure estimation is desired both in a hospital setting and in a home setting.

Traditional sphygmomanometers comprise an inflatable cuff configured to be worn around an arm of a subject. The cuff is inflated, so as to occlude the brachial artery, and then deflated, until the blood flow in the brachial artery is restored. The blood pressure of the subject wearing the cuff may be estimated with an oscillometric technique or an auscultatory technique. The former estimates the systolic and diastolic pressures based on the amplitude of the pressure oscillations recorded within the cuff during deflation or inflation, the latter estimates the systolic and diastolic pressures based on the Korotkoff sounds recorded with a stethoscope during the cuff deflation.

Finger and wrist sphygmomanometer have also been developed. However, these monitors are less accurate than upper-arm sphygmomanometer and, for this reason, the American Heart Association recommends not using them (Muntner, Paul, et al. "Measurement of blood pressure in humans: a scientific statement from the American Heart Association." Hypertension 73.5 (2019): e35-e66.). In fact, finger sphygmomanometers underestimate the blood pressure and are sensitive to background noise, whereas wrist blood pressure monitors are subjected to a lack of accuracy due to their tendency not to maintain the proper positioning. Moreover, the accuracy of blood pressure measurements at the wrist is highly affected by the position of the wrist relative to the heart.

Cuff-less blood pressure estimating are popular outside the clinical setting. These devices rely on the use of an optical sensor placed on the skin of a subject; the blood pressure of the subject is then estimated based on the signal collected from the optical sensor. Because of the inherent lack of accuracy of cuff-less devices, their use is not recommended in all those settings in which an accurate blood pressure estimation is needed, such as for instance in the clinical settings.

Cuff-based monitors configured to estimate the blood pressure at the arm level have high accuracy; however, they cannot provide regular blood pressure recording combined with optical sensor data. Such monitors cannot monitor patient health reliably and detect the need for blood pressure measurements. Moreover, conventional cuff-based monitors are not configured to measure relevant physiological parameters other than blood pressure.

The present invention aims to provide a device for non-invasive estimation of various physiological parameters of a subject that solves the problems of the prior art, namely, the device provides a continuous, or regular, and accurate blood pressure monitoring, while avoiding discomfort for the patient. More in particular, an object of the present invention is a wearable device capable of continuously or regularly and accurately measure a blood pressure of a wearer and signal when such measurements are required.

### SUMMARY

The present invention relates to a wearable device for blood pressure measurement comprising an armband with an inner surface and an outer surface, the armband comprising:
- an inflatable bladder;
- pumping means for inflating and deflating said bladder, the pumping means comprising a pump, a pipe in fluid communication with the inflatable bladder and a valve;
- a power supply device;
- a sensor array, the sensor array comprising at least one optical sensor and at least one pressure sensor;
- a processing unit;
wherein the armband comprises an opening configured to expose at least partially the optic sensor at the inner surface of the armband.

In one embodiment, the pressure sensor is replaced by an acoustic sensor.

In one embodiment, the power supply device comprises a built-in battery and a removable battery.

In one embodiment, the device according to the present invention further comprises a memory configured to store a list of events, the list of events comprising at least one time interval Δt; and the processing unit is configured to:
- retrieve, from the memory, the list of events and monitor the occurrence of said events;
- based on the monitoring, trigger the inflation and deflation of the inflatable bladder by means of the pumping means;
- receive the signal from the pressure sensor or the acoustic sensor during the inflation and/or deflation of the inflatable bladder;
- estimate a blood pressure value of the wearer based on the signal from the pressure sensor or the acoustic sensor.

In one embodiment, the processing unit is further configured to
- receive a signal from the optical sensor;
- execute a first algorithm configured to estimate a first blood pressure value of the wearer based on the signal from the optical sensor;
- execute a second algorithm configured to estimate a second blood pressure value of the wearer based on the signal from the pressure sensor or based on the signal from the acoustic sensor;
- estimate a set of parameters of the wearer based on the first and the second blood pressure values.

In one embodiment, the processing unit is configured to calibrate the first blood pressure value based on the estimated set of parameters of the wearer.

In one embodiment, the sensor array further comprises a temperature sensor and a heat flux sensor. In this embodiment, the processing unit may be further configured to receive a signal from the temperature sensor and a signal from the heat flux sensor and to execute a third algorithm, so as to estimate a core body temperature of the wearer based on said received signals.

In one embodiment, the sensor array further comprises at least one third sensor distinct from the aforementioned optical sensor, pressure sensor, temperature sensor and a heat flux sensor. Said at least one third sensor is selected from a group comprising, but not limited to: an ultrasound sensor; an accelerometer; a gyroscopic sensor; an electrical sensor; an optical sensor; a tomographic sensor; an acoustic sensor, a magnetometer; a humidity sensor; and the processing unit is further configured to receive a physiological signal from said third sensor. In one embodiment, the acoustic sensor may replace the pressure sensor.

The present invention also relates to a computer-implemented method for continuous monitoring of the blood pressure of a subject, the method comprising the following steps:
a) receiving, as input, a signal from an optic sensor;
b) estimating a first blood pressure value of the wearer by means of a first algorithm based on the signal received from the optical sensor;
c) monitoring the occurrence of events indicating that a calibration is required;
d) based on the monitoring, triggering the inflation and deflation of an inflatable bladder;
e) receiving, as input, a signal from a pressure sensor during deflation and/or inflation of the inflatable bladder;
f) estimating a second blood pressure value of the wearer by means of a second algorithm based on the signal received from the pressure sensor;
g) comparing the first blood pressure and the second blood pressure values;
h) based on the comparison, estimating a set of parameters of the wearer; and modifying the first algorithm.

In one embodiment, the method according to the present invention further comprises the following steps:
- estimating an alert value based on the signal received from the optic sensor;
- triggering the inflation and deflation of an inflatable bladder if the alert value is above a predefined threshold.

In one embodiment, the method according to the present invention further comprises a step of receiving, as input, a signal from a third sensor, said third sensor being distinct from the optical sensor and the pressure sensor, and in the estimation step b) a first blood pressure value of the wearer is estimated by means of a first algorithm based on the signal received from the optical sensor and/or on the signal received from the third sensor..

In one embodiment, the steps of the methods defined hereabove are preceded by a step of retrieving a set of parameters of the wearer from a memory. In this embodiment, in the estimation step b) a first blood pressure value of the wearer is estimated based on the signal received from the optic sensor and on the retrieved set of parameters.

In one embodiment, the steps b) and f) of estimating a first and a second blood pressure values are performed by means of a machine learning algorithm.

In one embodiment, the steps a) and b) are periodically repeated at a first repetition frequency and the steps c) to i) are periodically repeated at a second repetition frequency, the second repetition frequency being inferior to the first repetition frequency.

The present invention also relates to a computer program product for estimating the blood pressure of a subject, the computer program product comprising instructions which, when the program is executed by a computer, cause the computer to carry out the steps of the method as described hereabove.

The present invention also relates to a computer-readable storage medium comprising instructions which, when the program is executed by a computer, cause the computer to carry out the steps of the method as described hereabove.

### DETAILED DESCRIPTION

The following detailed description will be better understood when read in conjunction with the drawings. For the purpose of illustrating, the device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

The present invention relates to a wearable device for measuring the vital signs of a wearer. In a preferred embodiment, the present invention relates to a wearable device configured to provide a continuous, or regular, and accurate blood pressure monitoring.

More in particular, the present device is capable of: estimating a first blood pressure value based on the signal received from an optic sensor, detecting the occurrence of an event indicating that a calibration is required, triggering a cuff-based blood pressure estimation and calibrating the first blood pressure value based on the result of the cuff-based blood pressure estimation. Therefore, device allows to perform said calibration at an optimal frequency, thus providing a continuous, or regular, and accurate blood pressure monitoring, while avoiding discomfort for the patient.

The calibration may be launched when one or more predefined criteria are met, for instance, such criteria may relate to:
- an interval of time received as input, for instance introduced as input by a healthcare provider, or calculated by a processing unit;
- a signal metrics related to the signal collected from a sensor, preferably an optical sensor such as a photoplethysmographic (PPG) sensor;
- a physiological parameter of the wearer;
- a temporal evolution of the signal metrics;
- a temporal evolution of the physiological parameter of the wearer.

In one embodiment, the present invention relates to a wearable device for blood pressure measurement comprising an armband with an inner surface 13 and an outer surface 14, and the armband comprising:
- an inflatable bladder;
- pumping means for inflating and deflating said bladder, the pumping means comprising a pump 11, a pipe in fluid communication with the inflatable bladder and a valve 12;
- a power supply device 2;
- a processing unit;
- a sensor array, the sensor array comprising at least a first optical sensor and at least a second sensor, the second sensor being a pressure sensor 3 or an acoustic sensor;
wherein the armband comprises an opening 15 configured to expose at least partially the optic sensor at the inner surface 13 of the armband.

In one embodiment, the present device comprises an optical sensor and a pressure sensor. This embodiment allows to monitor the blood pressure of the wearer via an oscillometric technique.

In another embodiment, the pressure sensor may be replaced by an acoustic sensor. This embodiment allows to monitor the blood pressure of the wearer via an auscultatory technique.

In one embodiment, the wearable device further comprises a memory configured to store a list of events. The list of events may comprise conditions indicating that a calibration has to be performed.

The armband is configured to be worn around an upper arm of the subject.

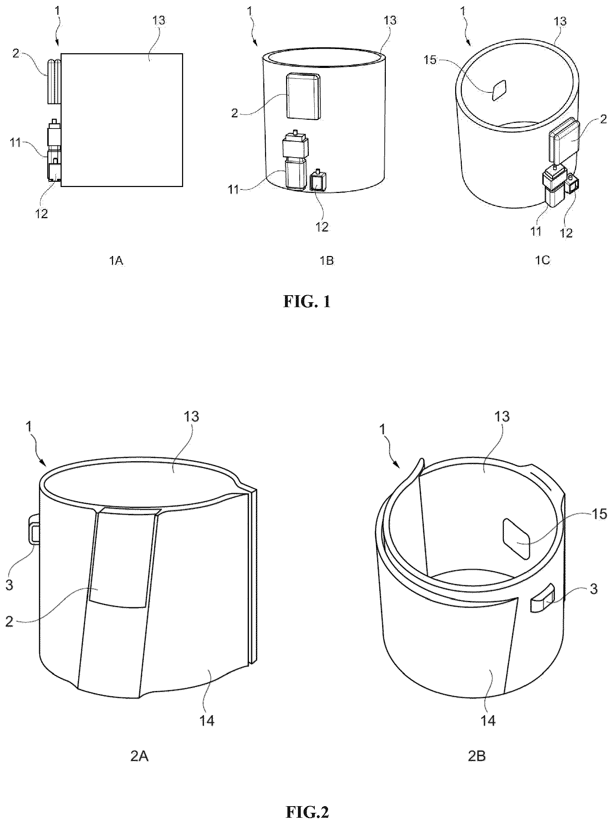

In one particular embodiment, the pumping means, the power supply device 2, the processing unit and the sensor array are buried inside the armband, between the inner surface 13 and the outer surface14. In this embodiment, only the optical sensor of the sensor array is exposed at the inner surface, and the other elements are not visible. One example of this embodiment is illustrated in figures 1A to 1C. In these figures, the outer surface 14 is not illustrated, so as to show the pump 11, the valve 12 and the power supply device 2, which otherwise would be hidden by the outer surface 14. In figure 1, the sensor array is not represented.

In another embodiment, one or more elements may be exposed at the outer surface 14 instead of being buried in the armband. For instance, the pressure sensor 3 may comprise a first portion buried inside the armband and a second portion exposed at the outer surface 14. One example of this embodiment is represented in figure 2A and figure 2B.

The power supply device 2 may also be buried inside the armband, partially exposed at the outer surface 14 or fully exposed at the outer surface 14. Figures 2A and 2B represent the device according to the present invention in a particular embodiment in which the power supply device 2 is fully exposed at the outer surface, so that it can be removed without dismantling the armband. This is particularly advantageous to easily replace or recharge the battery.

The blood pressure measured with the present device is indicative of the blood pressure in the arm arteries, such as the brachial or humeral artery, which is close to the blood pressure in the aorta. Therefore, the present invention allows to obtain a highly accurate and non-invasive blood pressure estimation.

The armband has a height and a length transverse to the height.

The armband length is long enough to cover the circumference of the upper arm of the subj ect.

In one embodiment, the armband length is comprised between 20 cm and 42 cm, preferably between 20 cm and 36 cm or between 20 cm and 30 cm.

The armband is high enough to accept at least an inflatable bladder, pumping means for inflating and deflating the bladder, a valve 12, a sensor array.

According to one embodiment, the armband height is comprised between 5 cm and 16 cm, preferably between 9 cm and 14 cm.

In one particular embodiment, the armband length is equal to 26 cm and the armband height is equal to 12 cm.

In one embodiment, the terms wearer, subject, user and patient have the same meaning.

In one embodiment, the armband is configured to be wrapped around the subject's upper arm and to be fastened with hook-and-loop attachment means.

In one embodiment, the armband is configured to be fastened with a buckle or an adhesive. In one embodiment, the armband is configured to be wrapped around the subject's upper arm and to be fastened with magnetic attachment means.

The device according to the present invention comprises an inflatable bladder. Said bladder is inflatable to a pressure that is high enough to uniformly restrict the blood flow in the subject's arm around which the armband is worn. In one embodiment, the bladder is inflatable up to a maximum inflation pressure superior to the pressure needed to occlude the brachial artery. In one embodiment, the maximum inflation pressure is equal to 250 mm Hg, preferably 260 mm Hg, 270 mm Hg or 280 mm Hg. In one embodiment, the bladder is inflatable up to a pressure of 300 mm Hg.

According to one embodiment, the inflatable bladder does not extend throughout the length of the armband. For instance, the inflatable bladder may extend for a length between 20 and 42 cm preferably between 22 cm and 36 cm., so as to cover for instance 80% of the patient arm.

According to one embodiment, the inflatable bladder may extend throughout the length of the armband.

In one embodiment, the inflatable bladder is made of resin, rubber, polyester, synthetic fiber, or a combination thereof. Preferably, the inflatable bladder is made of plastic fibers, TPU, PVC, or a combination thereof.

The present wearable device comprises pumping means for inflating and deflating the bladder.

Said pumping means comprising a pump 11, a pipe in fluid communication with the inflatable bladder and a valve 12.

In one embodiment, the pumping means comprise an electronic rolling air pump 11.

The device according to the present invention comprises a power supply device 2. Said power supply device 2 comprises a built-in battery and/or a removable battery.

The removable battery is configured to supply power to the wearable device of the present invention. In one embodiment, the removable battery is further configured to supply power to the built-in battery.

In one embodiment, the removable battery is rechargeable.

The built-in battery is configured to supply power to the wearable device in absence of a removable battery, for instance during the replacement of the removable battery.

This ensures uninterrupted power supply to the device, *i.e.* even when the removable battery is removed, thereby guaranteeing continuity of the blood pressure estimation and all vital signs monitoring.

In one embodiment, the built-in battery has capacity comprised between 10 mAh and 300 mAh, preferably between 30 mAh and 100 mAh. This embodiment ensures that the built-in battery duration is long enough to ensure the continuity of the functions performed by the device whilst the removable battery is not in place, for instance while the removable battery is being replaced or recharged. For instance, the continuity of the functions performed by the device whilst the removable battery is not in place may be ensured for at least 30 minutes; preferably for at least 40 minutes, 50 minutes or 60 minutes.

The armband of the present device comprises an inner surface 13, an outer surface 14 and an opening 15 configured to expose at least partially the optic sensor at the inner surface 13 of the armband.

In one embodiment, the armband comprises a slot. In this embodiment, the inflatable bladder may not extend throughout the length of the armband, and the slot may be in the armband portion that is not occupied by the bladder. In this embodiment, the power supply device 2 may be removably disposed within said slot, whereas the sensors of the sensor array are embedded inside the armband.

In one embodiment, the optical sensor is a single-wavelength photoplethysmographic (PPG) sensor comprising a light source and a light detector.

In one embodiment, the light source is an infrared LED. In one embodiment, the light source emits an infrared radiation from about 700 nm to about 1000 nm, preferably from about 900 nm to about 980 nm. In one preferred embodiment, the light source is configured to emit an infrared radiation of about 940 nm. In one embodiment, the infrared radiation wavelengths refer to the centroid wavelengths or to the peak wavelengths of the light emitters at their operating temperature. In one embodiment, the optical sensor array comprises a plurality of LEDs and photodetectors.

In one embodiment of the present invention, all sensors are integrated in a single sensor array.

In one embodiment, the sensors are not integrated in a single sensor array. For instance, the sensors may be separated by a predefined distance along the armband length and/or the armband height, so that when the wearable device is wrapped around an arm, the sensors are distributed around said arm.

In one embodiment, the sensor array comprises several optical sensors.

In one embodiment, the sensor array comprises three optical sensors. In this particular embodiment, the at least three optical sensors preferably comprise: at least one infrared LED, at least one green LED, at least one red LED. Preferably, said infrared LED, green LED and red LED are configured to emit a radiation having a radiation wavelength of 940 nm, 536 nm, and 655nm, respectively.

In one embodiment, the optical sensor further comprises a light filtering element configured to minimize the intensity of the light reflected from the skin surface without affecting the light reflected by subcutaneous tissues. In one embodiment, said light filter element comprises a first sheet of polarized glass placed on the light source of the optical sensor and a second sheet of polarized glass placed on the light detector of the optical sensor. The first and second sheet of polarized glass have of opposing polarization so as to filter out the polarized light reflected by the skin surface without affecting the depolarized light backscattered from the deeper skin layers.

In one embodiment, the optical sensor further comprises a light filtering element placed on the light detector of the optical sensor and configured to filter the wavelengths corresponding to a desired color component.

In one embodiment, the sensor array further comprises a temperature sensor and a heat flux sensor. In this embodiment, the processing unit may further be configured to receive a signal from the temperature sensor and a signal from the heat flux sensor and to estimate a core body temperature of the wearer based on said received signals.

In one embodiment, the processing unit is configured to:
- estimate at least one vital sign of the wearer;
- estimate the core body temperature of the wearer based on: a signal received from the temperature sensor, a signal received from the heat flux sensor, the at least one vital sign of the wearer.

The at least one vital sign of the wearer may be estimated based on the signal received from the optical sensor and/or the signal from the pressure sensor 3. The at least one vital sign may be for instance: the heart rate (HR), the respiratory rate (RR), the oxygen saturation (Sp02), or any vital sign of the wearer.

The estimation of the core body temperature of the wearer may be performed by a third algorithm distinct from the first and second algorithms. In one embodiment, said third algorithm is a machine learning algorithm.

In one embodiment, the sensor array further comprises at least one additional sensor which is distinct from the optical sensor and the pressure sensor 3. For instance, in one embodiment the sensor array may comprise a temperature sensor and heat flux sensor.

This embodiment is particularly advantageous to estimate a core body temperature of the wearer.

The at least one additional sensor may be selected from a list including, but not limited to: an ultrasound sensor; an accelerometer; a gyroscopic sensor; an electrical sensor, such as for instance an electrical sensor comprising electrodes; a tomographic sensor; an acoustic sensor; a magnetometer; a humidity sensor; an impedance sensor. For instance, the sensor array may comprise a tomographic sensor or an acoustic sensor. In this particular example, the processing unit may estimate an arterial noise based on the signal received from the tomographic sensor or the acoustic sensor, and it may characterize the blood flow based on said arterial noise.

In one embodiment, the additional sensor is a sweat sensor. Said sweat sensor may be an impedance-based sensor comprising electrodes configured to emit and receive a small intensity current.

In one embodiment, the additional sensor is an optical sensor. In this embodiment, the sensor array comprises a first optical sensor, and one or more additional optical sensors distinct from said first optical sensor. The processing unit may be configured to estimate a first blood pressure value of the wearer based on the signal from said first optical sensor; and to derive a plurality of physiological parameters of the wearer, such as for example the blood oxygen saturation, based on the signals received from the additional optical sensors. In one embodiment, the light sources of the additional optical sensors are configured to emit light radiations having different wavelengths.

In one embodiment, the sensor array is entirely housed inside the armband, i.e. it is positioned within the inner surface 13 and the outer surface 14 of the armband. The inner surface 13 of the armband comprises an opening 15, configured to expose at least the optical sensor of the sensor array.

In one embodiment, the device according to the present invention further comprises a memory configured to store a list of events.

In one embodiment, the list of events comprises at least one time interval Δt.

In one embodiment, the list of events comprises at least one signal metric and a range of values associated with said signal metric.

The memory may further be configured to store the most recent first blood pressure values of the wearer. For instance, the memory may store at least the last 480, 240, 120, 60, or 30 first blood pressure values of the wearer.

The present wearable device further comprises a processing unit that is capable of triggering the inflation or deflation of the inflatable bladder, by opening or closing the valve 12 of the pumping means.

The processing unit is further configured to estimate physiological parameters of the wearer based on the signal received from the sensors of the sensor array. The processing unit may be configured to execute two or more algorithms to perform said estimation.

More in particular, the processing unit is configured to receive the signal from the optical sensor and estimate a first blood pressure value based on the signal received from the optical sensor. The estimation of the first blood pressure value is performed by means of a first algorithm executed by the processing unit.

The processing unit is further configured to retrieve the list of events stored in the memory, and to monitor the occurrence of said events. When the occurrence of an event from the list of events is detected, the processing unit may trigger the inflation and the deflation of the inflatable bladder. During the inflation and/or deflation of the bladder, the processing unit receives the signal from the pressure sensor 3 of the sensor array.

A second algorithm is executed by the processing unit to estimate a second blood pressure value of the wearer based on the signal from the pressure sensor 3.

Since the second blood pressure value is obtained with a cuff-based method, it is highly accurate.

Based on said first and second blood pressure values, the processing unit is capable of deriving a set of parameters of the wearer. Advantageously, the set of parameters of the wearer can be used to calibrate the first blood pressure value.

In fact, the since the first blood pressure value is estimated based on the signal received from an optical sensor, a calibration is needed in order to ensure that the first blood pressure value is accurate.

In one embodiment, the present device allows to automatically perform periodical calibrations of the first blood pressure value, at regular time interval. Therefore, the list of events may comprise a time interval that is indicative of an optimal calibration frequency.

In this embodiment, the processing unit is configured to trigger the inflation and deflation of the inflatable bladder at the end of the time interval. Therefore, the triggering is repeated at a fixed repetition frequency.

In this embodiment, the inflation and deflation of the inflatable bladder may be triggered independently of the characteristics of the signal received from the optic sensor.

In one embodiment, the time interval is comprised between 0.25 hours and 8 hours.

The processing unit may trigger the inflation and deflation of the inflatable bladder based on the signal received from one or more sensors. For instance, the processing unit may modify the time interval based on said signal received from one or more sensors. Alternatively, the processing unit may trigger the inflation and deflation of the inflatable bladder based on the signal received from one or more sensors, without modifying the time interval. In this particular embodiment, if the signal received from one or more sensors meet a predefined criterion during the time interval the processing unit trigger the inflation and deflation of the inflatable bladder, then it restarts the same time interval.

In one embodiment, the processing unit may trigger the inflation and deflation of the inflatable bladder based on the signal received from the optical sensor.

In one embodiment, the sensor array may comprise a temperature sensor, and the processing unit may increase or decrease the duration of the time interval based on the temperature of the wearer.

In one embodiment, the sensor array may comprise an accelerometer; the processing unit may be capable of detecting an activity level of the wearer based on the signal from the accelerator, and it may increase or decrease the duration of the time interval based on said activity level.

In one embodiment, the time interval is comprised between 6 hours and 12 hours; preferably between 6 hours and 10 hours. This embodiment is advantageous when a lower calibration frequency is required, for instance when the wearer is a child.

In one embodiment, the present device allows to launch an "on demand" calibration of the first blood pressure value. This embodiment is advantageous since in some cases, the wearer or a healthcare specialist may need to launch the inflation and deflation of the bladder, without the occurrence of an event indicating that the calibration is required.

In this embodiment, the wearable device further comprises an acquisition module configured to receive as input a request of calibration.

This embodiment is particularly advantageous when the wearer or a healthcare specialist need to: estimate the second blood pressure value based on the signal from the pressure sensor 3, and/or to estimate a set of parameters of the wearer without the occurrence of an event indicating that the calibration is required.

In one embodiment, the list of events comprises at least one signal metric and a range of values associated with said signal metric. In this embodiment, the processing unit is configured to:
- calculate a value of the at least one metric related to the signal received from a first sensor, such for instance from the optical sensor;
- compare the calculated value of the at least one metric with the threshold or the range of values in the list of events;
- based on the comparison, trigger the inflation and deflation of the inflatable bladder by means of the pumping means.

The processing unit receives the signal from the pressure sensor 3 during said inflation and/or deflation of the inflatable bladder, and it is configured to:
- execute a first algorithm configured to estimate a first blood pressure value of the wearer based on the signal from the first sensor and a second algorithm configured to estimate a second blood pressure value of the wearer based on the signal from the pressure sensor 3;
- estimate a set of parameters of the wearer based on the first and the second blood pressure values.

In this embodiment, the processing unit triggers the inflation and deflation of the inflatable bladder when a calculated signal metric is outside the range of values that is associated with said signal metric in the list of events.

Therefore, in this embodiment, the first blood pressure value may be calibrated at a variable calibration frequency. Since the signal metrics related to the optical sensor may be modified in response of a change in the wearer's status, this embodiment is particularly advantageous since it allows to increase or decrease the calibration frequency based on in the wearer's status.

For instance, the at least one metrics may be selected from a group comprising, but not limited to: maximum, minimum, slope, derivative, mean value, median value, coefficient of variation, signal-to-noise ratio, or a combination thereof, such as a linear or polynomial combination. For instance, the at least one signal metric may be a difference between a signal metric measured at the time interval t+Δt and a signal metric measured at a time t.

In one embodiment, the sensor array of the present device comprises at least on additional sensor distinct from the optical and pressure sensor 3, and the signal metric is calculated based on the signal from said additional sensor.

In one embodiment, the list of events in the memory comprises at least one time interval and at least one signal metric. In this embodiment, the processing unit may trigger the inflation and deflation of the inflatable bladder when a combination of events occurs. For instance, the processing unit may monitor the at least one signal metric during a first time interval, the duration of the first time interval being retrieved from the list of events in the memory. the processing unit may be configured to:
- if, during the first time interval, the signal metric is outside the range of values associated with said signal metric in the list of events,
   - prematurely end the first time interval;
   - trigger the inflation and deflation of the inflatable bladder;
   - start a second time interval, the duration of the second time interval being retrieved from the list of events in the memory;
- if, during the first time interval, the signal metric is not outside the range of values associated with said signal metric in the list of events,
   - trigger the inflation and deflation of the inflatable bladder at the end of the first time interval; and
   - start a second time interval, the duration of the second time interval being retrieved from the list of events in the memory.

In one embodiment, the present device comprises an acquisition module configured to receive, as input, the wearer's data. The wearer's data may comprise, for instance: gender, age, weight. In one embodiment, the wearer is a patient and the acquisition module may be configured to extract the wearer's data from an electronic medical record of the patient. In one embodiment, the device may comprise a memory that is configured to store said wearer's data.

In one embodiment, the acquisition module is configured to receive as input a request of calibration.

In one embodiment, the acquisition module is further configured to receive data from a continuous or regular glucose monitoring device. Optionally, the power supply device 2 may be configured to supply power to the continuous or regular glucose monitoring device.

In one embodiment, the present device comprises a transmission module. The transmission module may be configured to transmit parameters estimated by the processing unit, such as for instance: the first blood pressure value and the second blood pressure value, a set of parameters of the wearer. Preferably, the transmission module is a Bluetooth-based transmission module.

In one embodiment, the algorithms executed by the processing unit are configured to estimate physiological parameters of the wearer based on the signal received from the sensors of the sensor array and on the wearer's data.

Of note, the present invention avoids the use of multiple optical sensor placed at different locations, which would cause discomfort to the subject. Moreover, the present invention does not require the subject to displace the optical sensor, hence it can be used to estimate the blood pressure of a subject that could not execute said displacement, for instance a subject having reduced mobility, immobility or pain; a subject that is asleep, under anesthesia or in a coma; a subject that cannot understand the request of displacing the sensor, such as an infant, or a person with impaired cognitive abilities.

For instance, the processing unit may receive sound data from the acoustic sensor, and estimate a physiological parameter of the wearer based on the received sound data. For instance, the received sound data may be indicative of a turbulent blood flow or a laminar blood flow.

The present invention allows to estimate a set of parameters of the wearer. This set of parameters is used to calibrate the first blood pressure values.

In one embodiment, the estimated set of parameters of the wearer comprises parameters representative of hemodynamic property, mechanical property or geometrical property related to the arterial blood flow. For instance, the estimated set of parameters may be selected from a group comprising, but not limited to: arterial compliance, arterial cross-sectional area, peripheral resistance, arterial stiffness, arterial wall thickness, blood flow, blood pressure, blood vessel elasticity index, cardiac output, elastic modulus of artery, heart rate, plasma volume, stroke volume, vessel length to radius ratio.

In one embodiment, the estimated set of parameters of the wearer comprises algorithmic parameters. By algorithmic parameter, it is meant a parameter derived by means of an algorithm which is related to one or more physiological parameters, but it does not necessarily represent a measure of a physiological parameter. For instance, the estimated set of parameters of the wearer comprises feature parameters developed for machine learning purposes.

In one embodiment, the set of parameters comprises a histogram statistic. Said histogram statistics may be selected from a group comprising, but not limited to: 10^{th} percentile, 25^{th} percentile, 75^{th} percentile, 90^{th} percentile, entropy, intensity range, intensity variance, intensity standard deviation, kurtosis, mean absolute deviation, minimum intensity, maximum intensity, mean intensity, median intensity, root mean square, skewness, standard deviation, uniformity, variance.

The present invention also relates to a computer-implemented method for blood pressure estimation of a subject wearing an armband comprising an inflatable bladder, a first sensor and a pressure sensor 3, the method comprising a step of receiving, as input, a signal from the first sensor and a step of estimating, by means of a first algorithm, a first physiological parameter of the wearer based on the signal received from the first sensor.

In one embodiment, the method further comprises a step of monitoring the occurrence of an event indicating that a calibration is required and a step of triggering the inflation and deflation of an inflatable bladder, based on said monitoring. During said inflation and/or deflation, a cuff-based blood pressure estimation can be performed.

The event indicating that a calibration is required may comprise a criterion related to the signal received from the first sensor. This embodiment allows to launch a cuff-based blood pressure measurement based on the signal collected from the first sensor, preferably an optic sensor. For instance, a calibration may be needed when a signal metric is abnormal, or when the temporal evolution over a predefined period of time is abnormal.

In one embodiment, the first sensor is an optic sensor and the first physiological parameter is a blood pressure.

In one embodiment, the method according to the present invention comprises a step of monitoring the occurrence of events indicating that a calibration is required. Based on the monitoring, the inflation and deflation of an inflatable bladder is triggered. For instance, the list of events may comprise criteria related to the signal received from a sensor.

During the inflation and/or the deflation of the inflatable bladder, a signal from a pressure sensor 3 is received and a blood pressure value of the wearer is estimated based on said signal.

The present invention also relates to a method for estimating the blood pressure of a subject wearing an armband comprising an inflatable bladder, a first sensor, which is preferably an optical sensor, and a second sensor, which is preferably a pressure sensor 3. This method comprises the following step: estimating a first blood pressure of the subject based on the signal received from the first sensor; estimating a second blood pressure of a subject based on the signal received from the second sensor; comparing the first blood pressure and the second blood pressure values; based on the comparison, estimating a set of parameters of the wearer.

The present invention also relates to a method for blood pressure estimation of a subject wearing an armband comprising an inflatable bladder, an optical sensor and a pressure sensor 3, the method being implemented by a processing unit, and comprising the following steps:
a) receiving, as input, a signal from an optic sensor;
b) estimating a first blood pressure value of the wearer by means of a first algorithm based on the signal received from the optical sensor;
c) monitoring the occurrence of events indicating that a calibration is required;
d) based on the monitoring, triggering the inflation and deflation of an inflatable bladder;
e) receiving, as input, a signal from a pressure sensor 3 during deflation and/or inflation of the inflatable bladder;
f) estimating a second blood pressure value of the wearer by means of a second algorithm based on the signal received from the pressure sensor 3;
g) comparing the first blood pressure and the second blood pressure values;
h) based on the comparison, estimating a set of parameters of the wearer;
i) based on the set of parameters of the wearer, modifying the first algorithm.

In one embodiment, the step b) of estimating a first blood pressure value is performed by a machine learning algorithm. In one embodiment, the first blood pressure value is calculated based on the signal from the optic sensor, via a step of decomposition into features and selection of applicable features. Advantageously, this embodiment allows to accurately estimate the blood pressure of the wearer while minimizing the number of required signals: in fact, the first blood pressure values of the wearer are estimated based on the signal from the optical sensor, and periodically calibrated based on the signal from the pressure sensor 3, the frequency of the periodic calibrations being determined by the criteria stored in a list of events in a memory. This embodiment allows to estimate a first blood pressure based on a single optic sensor, thereby avoiding the use of multiple optic sensor placed at different location, which is expensive and may cause discomfort to the user.

In another embodiment, the step b) of estimating a first blood pressure comprises:
- calculating at least one blood pressure indicator based on the signal from the optic sensor,
- estimating a first blood pressure of the wearer based of the wearer based on said at least one blood pressure indicator.

The blood pressure indicator may be calculated based on several signals. For instance, it may be calculated based on a first signal from the optical sensor and a second signal from an ECG or PPG sensor. In this embodiment, the blood pressure indicator may be, for example: a pulse arrival time, a pulse transit time, a central pulse transit time, a peripheral pulse transit time.

The present method allows to periodically calibrate the first blood pressure values, based on the set of parameters of the wearer. The calibration frequency may be constant or variable, depending on the occurrence of events indicating that a calibration is required.

In one embodiment, the steps a) to i) are periodically repeated. After each repetition, an updated set of parameters of the wearer may be stored in the memory and replace the previous set of parameters of the wearer.

In one embodiment, the steps a) to b) are periodically repeated at a first repetition frequency. In one embodiment, the first repetition frequency is comprised between 0.0005 s⁻¹ and 0.07 s⁻¹, preferably between 0.001 s⁻¹ and 0.016 s⁻¹.

In one embodiment, the steps c) to i) are periodically repeated at a second repetition frequency, the second repetition frequency being inferior to the first repetition frequency. The second repetition frequency may be constant or variable. For instance, if the event indicating that a calibration is required comprises a time-based condition, the second repetition frequency may be constant. For instance, if the event indicating that a calibration is required comprises a sensor-based condition, the second repetition frequency may be variable.

In one embodiment, the terms "calibration frequency" and "second repetition frequency" have the same meaning.

The present invention also relates to a computer program product for blood pressure estimation of a subject wearing an armband comprising an inflatable bladder, a first sensor, preferably an optical sensor, and a second sensor, preferably a pressure sensor 3. The computer program product is capable of estimating a first blood pressure of the subject based on the signal received from the first sensor; estimating a second blood pressure of a subject based on the signal received from the second sensor; comparing the first blood pressure and the second blood pressure values; based on the comparison, estimating a set of parameters of the wearer.

In one embodiment, the computer program product is capable of estimating the first blood pressure of a subject based on the signal received from the first sensor and from a signal received from a third sensor.

The computer program product to perform the method as described above may be written as computer programs, code segments, instructions or any combination thereof, for individually or collectively instructing or configuring the processor or computer to operate as a machine or special-purpose computer to perform the operations performed by hardware components. In one example, the computer program product includes machine code that is directly executed by a processor or a computer, such as machine code produced by a compiler. In another example, the computer program product includes higher-level code that is executed by a processor or a computer using an interpreter. Programmers of ordinary skill in the art can readily write the instructions or software based on the block diagrams and the flow charts illustrated in the drawings and the corresponding descriptions in the specification, which disclose algorithms for performing the operations of the method as described above.

The present invention also relates to a computer-readable storage medium comprising instructions which, when the program is executed by a computer, cause the computer to carry out the steps of the method according to any one of the embodiments described hereabove.

According to one embodiment, the computer-readable storage medium is a non-transitory computer-readable storage medium.

Computer programs implementing the method of the present embodiments can commonly be distributed to users on a distribution computer-readable storage medium such as, but not limited to, an SD card, an external storage device, a microchip, a flash memory device, a portable hard drive and software websites. From the distribution medium, the computer programs can be copied to a hard disk or a similar intermediate storage medium.

The computer programs can be run by loading the computer instructions either from their distribution medium or their intermediate storage medium into the execution memory of the computer, configuring the computer to act in accordance with the method of this invention. All these operations are well- known to those skilled in the art of computer systems.

The instructions or software to control a processor or computer to implement the hardware components and perform the methods as described above, and any associated data, data files, and data structures, are recorded, stored, or fixed in or on one or more non-transitory computer-readable storage media. Examples of a non-transitory computer-readable storage medium include read-only memory (ROM), random-access memory (RAM), flash memory, CD- ROMs, CD- Rs, CD+Rs, CD- RWs, CD+RWs, DVD-ROMs, DVD- Rs, DVD+Rs, DVD- RWs, DVD+RWs, DVD- RAMs, BD- ROMs, BD- Rs, BD- R LTHs, BD- REs, magnetic tapes, floppy disks, magneto-optical data storage devices, optical data storage devices, hard disks, solid-state disks, and any device known to one of ordinary skill in the art that is capable of storing the instructions or software and any associated data, data files, and data structures in a non-transitory manner and providing the instructions or software and any associated data, data files, and data structures to a processor or computer so that the processor or computer can execute the instructions. In one example, the instructions or software and any associated data, data files, and data structures are distributed over network-coupled computer systems so that the instructions and software and any associated data, data files, and data structures are stored, accessed, and executed in a distributed fashion by the processor or computer.

While various embodiments have been described and illustrated, the detailed description is not to be construed as being limited hereto. Various modifications can be made to the embodiments by those skilled in the art without departing from the true spirit and scope of the disclosure as defined by the claims.

### BRIEF DESCRIPTION OF THE DRAWINGS

**Figure 1** represents the device of the present invention according to a first embodiment in which the power supply device 2, the pump 11, the valve 12, and the pressure sensor 3 are buried in the armband, i.e. they are positioned between the inner surface 13 and the outer surface, and wherein the power supply device 2 and the opening 15 are spaced apart along the armband length so that, when the device is wrapped around an arm, said power supply device 2 and the opening 15 are diametrically opposed with respect to the arm. In figure 1, the outer surface is not illustrated for intelligibility purpose, so as to show the elements buried in the armband.
**Figure 1A** is a side view of the present device according to said first embodiment.
**Figure 1B** is a perspective view of the present device according to said first embodiment.
**Figure 1C** is a perspective view of the present device according to said first embodiment.
**Figure 2** represents the device of the present invention according to a second embodiment in which the pumping means are buried in the armband, whereas the power supply device 2, and the pressure sensor 3 are exposed at the outer surface 14, and wherein the opening 15 and the power supply device 2 are not spaced apart along the armband length; therefore, they are on the same side of an arm when the device is wrapped around said arm.
**Figure 2A** is a perspective view representing the device of the present invention according to the second embodiment.
**Figure 2B** is a perspective view representing the device of the present invention according to the second embodiment.

## Claims

1. A wearable device for blood pressure measurement comprising an armband with an inner surface (13) and an outer surface (14), the armband comprising:
- an inflatable bladder;
- pumping means for inflating and deflating said bladder, the pumping means comprising a pump (11), a pipe in fluid communication with the inflatable bladder and a valve (12);
- a power supply device (2);
- a sensor array, the sensor array comprising at least one optical sensor and at least one pressure sensor (3);
- a processing unit;
wherein the armband comprises an opening (15) configured to expose at least partially the optical sensor at the inner surface (13) of the armband.

2. The device according to claim **1**, wherein the power supply device (2) comprises a built-in battery and a removable battery.

3. The device according to claim **1** or claim **2**, further comprising a memory configured to store a list of events, the list of events comprising at least one time interval Δt, wherein processing unit is configured to:
- retrieve, from the memory, the list of events and monitor the occurrence of said events;
- based on the monitoring, trigger the inflation and deflation of the inflatable bladder by means of the pumping means;
- receive the signal from the pressure sensor (3) during the inflation and/or deflation of the inflatable bladder;
- estimate a blood pressure value of the wearer based on the signal from the pressure sensor (3).

4. The device according to claim **3**, wherein processing unit is further configured to
- receive a signal from the optical sensor;
- execute a first algorithm configured to estimate a first blood pressure value of the wearer based on the signal from the optical sensor;
- execute a second algorithm configured to estimate a second blood pressure value of the wearer based on the signal from the pressure sensor (3);
- estimate a set of parameters of the wearer based on the first and the second blood pressure values.

5. The device according to claim **4**, wherein the processing unit is configured to calibrate the first blood pressure value based on the estimated set of parameters of the wearer.

6. The device according to any of claims **1** to **5**, wherein the sensor array further comprises a temperature sensor and a heat flux sensor; and wherein the processing unit is further configured to receive a signal from the temperature sensor and a signal from the heat flux sensor and to execute a third algorithm to estimate a core body temperature of the wearer based on said received signals.

7. The device according to any of claims **1** to **6**, wherein the sensor array further comprises at least one third sensor selected from a group comprising: an ultrasound sensor, a accelerometer, a gyroscopic sensor, an electrical sensor, an optical sensor, a tomographic sensor, an acoustic sensor, whereby the acoustic sensor may replace the pressure sensor, a magnetometer, a humidity sensor; and wherein the processing unit is further configured to receive a signal from said third sensor and to estimate a physiological parameter based on said received signal.

8. A computer-implemented method for continuous or regular monitoring of the blood pressure of a subject, the method comprising the following steps:
a) receiving, as input, a signal from an optic sensor;
b) estimating a first blood pressure value of the wearer by means of a first algorithm based on the signal received from the optical sensor;
c) monitoring the occurrence of events indicating that a calibration is required;
d) based on the monitoring, triggering the inflation and deflation of an inflatable bladder;
e) receiving, as input, a signal from a pressure sensor (3) during deflation and/or inflation of the inflatable bladder;
f) estimating a second blood pressure value of the wearer by means of a second algorithm based on the signal received from the pressure sensor (3);
g) comparing the first blood pressure and the second blood pressure values;
h) based on the comparison, estimating a set of parameters of the wearer; and modifying the first algorithm.

9. The method according to claim **8**, further comprising the following steps:
- estimating an alert value based on the signal received from the optic sensor;
- triggering the inflation and deflation of an inflatable bladder if the alert value is above a predefined threshold.

10. The method according to claim **8** or claim **9**, further comprising a step of receiving, as input, a signal from a third sensor, said third sensor being distinct from the optical sensor and the pressure sensor (3), and wherein in the estimation step b) a first blood pressure value of the wearer is estimated by means of a first algorithm based on the signal received from the optical sensor and/or on the signal received from the third sensor.

11. The method according to any of claims **8** to **10**, wherein the steps of the methods are preceded by a step of retrieving a set of parameters of the wearer from a memory; and wherein in the estimation step b) a first blood pressure value of the wearer is estimated based on the signal received from the optic sensor and on the retrieved set of parameters.

12. The method according to any of claims **8** to **11**, wherein the steps b), f) of estimating a first and a second blood pressure values are performed by means of a machine learning algorithm.

13. The method according to any of claims **8** to **12**, wherein the steps a) and b) are periodically repeated at a first repetition frequency and the steps c) to i) are periodically repeated at a second repetition frequency, the second repetition frequency being inferior to the first repetition frequency.

14. A computer program product for estimating the blood pressure of a subject, the computer program product comprising instructions which, when the program is executed by a computer, cause the computer to carry out the steps of the method according to any of claims **8** to **13.**

15. A computer-readable storage medium comprising instructions which, when the program is executed by a computer, cause the computer to carry out the steps of the method according to any of claims **8** to **13.**
